# EUROPÄISCHE PATENTANMELDUNG

(11) **EP 2 540 725 A1**
(43) Veröffentlichungstag der Anmeldung: **02.01.2013**
(21) Anmeldenummer: 12185396.4
(22) Anmeldetag: 30.04.2007
(51) Int. Cl.: C07D 473/04, A61K 31/522, A61P 3/10

(54) **Polymorphe von 1-((4-Methyl-chinazolin-2-yl)methyl)-3-methyl-7-(2-butin-1-yl)-8-(3-(R)-amino-piperidin-1-yl)xanthin**

(30) Priorität: 04.05.2006 EP 06009202
(62) Teilanmeldung aus: 07728655.7
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Sieger, Peter, 55216 INGELHEIM AM RHEIN (DE); Kemmer, Dirk, 55216 INGELHEIM AM RHEIN (DE); Kohlbauer, Peter, 55216 INGELHEIM AM RHEIN (DE); Nicola, Thomas, 55216 INGELHEIM AM RHEIN (DE); Renz, Martin, 55216 INGELHEIM AM RHEIN (DE)
(74) Vertreter: Simon, Elke Anna Maria

(57) **Zusammenfassung**

Die Erfindung betrifft polymophe Kristallmodifikationen eines DPP-IV Inhibitors, 1-[(4-Methyl-chinazolin-2-yl)methyl]-3-methyl-7-(2-butin-1-yl)-8-(3-(R)- amino-piperidin-1-yl)-xanthin deren Herstellung und deren Verwendung zur Herstellung eines Arzneimittel.

## Beschreibung

Die Erfindung betrifft polymorphe Kristallmodifikationen eines DPP-IV inhibitors, deren Herstellung und deren Verwendung zur Herstellung eines Arzneimittel.

Das Enzym DPP-IV, auch bekannt unter der Bezeichnung CD26, ist eine Serinprotease, die bei Proteinen mit einem Prolin oder Alanin Rest am N-terminalen Ende die Abspaltung von Dipeptiden begünstigt. DPP-IV Inhibitoren beeinflussen dadurch den Plasmaspiegel bioaktiver Peptide einschließlich des Peptids GLP-1. Verbindungen diese Typs sind interesant zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, Prädiabetes, oder Verminderung der Glukosetoleranz.

In WO 2004/018468 werden DPP-IV Inhibitoren mit wertvollen pharmakologischen Eigenschaften beschrieben. Ein Beispiel der dort offenbarten Inhibitoren ist 1-[(4-Methyl-chinazolin-2-yl)methyl]-3-methyl-7-(2-butin-1-yl)-8-(3-(R)-amino-piperidin-1-yl)-xanthin.

Im Rahmen der vorliegenden Erfindung wurde gefunden, dass 1-[(4-Methyl-chinazolin-2-yl)methyl]-3-methyl-7 -(2-butin-1-yl)-8-(3-(R)-amino-piperidin-1-yl)-xanthin verschiedene polymorphe Kristallmodifikationen annehmen kann und dass die in WO 2004/018468 hergestellte Verbindung bei Raumtermperatur als eine Mischung zweier enantiotroper Polymorphe vorliegt. Die Temperatur bei der sich die beiden Polymorphe ineinander umwandeln liegt bei 25±15°C (siehe Abbilding 1 und 2).

Die reine Hochtemperaturform (Polymorph A), die durch Erwärmen des Gemisches auf Temperaturen >40°C erhalten werden kann, schmilzt bei 206±3 °C. Im Röntgenpulverdiagramm (siehe Abbilding 3) zeigt diese Form charakteristische Reflexe bei folgenden d-Werten: 11.49 Å, 7.60 Å, 7.15 Å, 3.86 Å, 3.54 Å und 3.47 Å (siehe auch Tabelle 1 und 2).

Herstellen lässt sich wasserfreies Polymorph A dadurch, dass man
(a) 1-[(4-Methyl-chinazolin-2-yl)methyl]-3-methyl-7-(2-butin-1-yl)-8-(3-(R)-aminopiperidin-1-yl)-xanthin in absolutem Ethanol zum Rückfluß erhitzt und gegebenenfalls filtriert,
(b) die heiße Lösung bzw. das heiße Filtrat bis zum Einsetzen der Kristallisation abkühlt,
(c) mit einem Lösungsmittel wie tert.-Butylmethylether verdünnt,
(d) das Lösungsmittelgemisch absaugt und
(e) das Polymorph A bei 45°C im Vakuum trocknet.

Die Tieftemperaturform (Polymorph B) wird durch Abkühlen auf Temperaturen <10 °C erhalten. Im Röntgenpulverdiagramm (siehe Abbildung 4) zeigt diese Form charakteristische Reflexe bei folgenden d-Werten: 11.25 Å, 9.32 Å, 7.46 Å, 6.98 Å und 3.77 Å (siehe auch Tabelle 3 und 4).

Herstellen lässt sich wasserfreies Polymorph B dadurch, dass man
(a) 1-[(4-Methyl-chinazolin-2-yl)methyl]-3-methyl-7-(2-butin-1-yl)-8-(3-(R)-aminopiperidin-1-yl)-xanthin in absolutem Ethanol löst und zum Rückfluß erhitzt und gegebenenfalls filttriert,
(b) die heiße Lösung bzw. das heiße Filtrat für die Kristallisation unter eine Temperatur von 10°C abkühlt,
(c) mit einem Lösungsmittel wie tert.-Butylmethylether verdünnt,
(d) das Lösungsmittelgemisch absaugt und
(e) das Polymorph bei einer Temperatur von weniger als 10°C unter Vakuum trocknet.

Ein anderes Polymorph (Polymorph C) zeigt im Röntgenpulverdiagramm (siehe Abbildung 5) charakteristische Refelxe bei folgenden d-Werten: 12.90 Å, 11.10 Å, 6.44 Å, 3.93 Å und 3.74 Å (siehe auch Tabelle 5.

Man erhält Polymorph C, wenn man
(a) 1-[(4-Methyl-chinazolin-2-yl)methyl]-3-methyl-7-(2-butin-1-yl)-8-(3-(R)-amino-piperidin-1-yl)-xanthin in Methanol löst und zum Rückfluß erhitzt und gegebenenfalls in Gegenwart von Aktivkohle filtriert,
(b) die methanolische Lösung auf eine Temperatur von 40-60°C abkühlt,
(c) mit einem Lösungsmittel wie tert.-Butylmethylether oder Diisopropylether versetzt,
(d) die resultierende Suspension zunächst langsam auf 15-25°C und später dann auf 0-5°C abkühlt,
(e) die entstandenen Kristalle absaugt absaugt und nochmals mit tert.-Butylmethylether oder Diisopropylether nachwäscht und
(f) die so erhaltenen Kristalle bei einer Temperatur von 70°C im Vakuumtrockenschrank trocknet.

Ein weiteres Polymorph (Polymorph D) schmilzt bei 150±3 °C. Dieses Polymorph erhält man, wenn Polymorph C auf eine Temperatur von 30-100°C erwärmt bzw. bei dieser Temperatur trocknet.

Schliesslich gibt es auch Polymorph E, das bei einer Temperatur von 175±3 °C schmilzt. Wasserfreies Polymorph E entsteht, wenn Polymorph D geschmolzen wird. Bei weiterer Erwärmung kristallisiert aus der Schmelze Polymorph E aus.

Die so erhaltenen Polymorphe können ebenso wie die in WO 2004/018468 beschriebene Polymorphenmischung der beiden Polymorphe A und B zur Herstellung eines Arzneimittels verwendet werden, das zur Behandlung von Patienten mit Diabetes mellitus Typ I und Typ II, Prädiabetes oder verminderter Glukosetoleranz, mit rheumatoider Arthritis, Adipositas, oder durch Calcitonin verursachter Osteoporose, sowie von Patienten, bei denen eine Allograft Transplantation vorgenommen wurde. Diese Arzneimittel enthalten neben einem oder mehreren inerten Trägerstoffen wenigstens 0,1% bis 0,5%, vorzugsweise wenigstens 0,5% bis 1,5% und besonders bevorzugt wenigstens 1 % bis 3% eines der Polymorphe A, B, oder C

Die folgenden Beispiele sollen die Erfindung näher erläutern.

### Beispiel 1 Kristallisation des Polymorphs A

1-[(4-Methyl-chinazolin-2-yl)methyl]-3-methyl-7-(2-butin-1-yl)-8-(3-(R)-amino-piperidin-1-yl)-xanthin Rohsubstanz wird mit der 5-fachen Menge an absolutem Ethanol zum Rückfluß erhitzt und die heiße Lösung über Aktiv-Kohle klarfiltriert. Nach Abkühlen des Filtrates auf 20°C und Einsetzen der Kristallisation wird mit tert.-Butylmethylether auf das doppelte Volumen verdünnt. Anschliessend wird die Suspension wird auf 2°C abgekühlt, 2 Stunden nachgerührt, abgesaugt und im Vakuumtrockenschrank bei 45°C getrocknet.

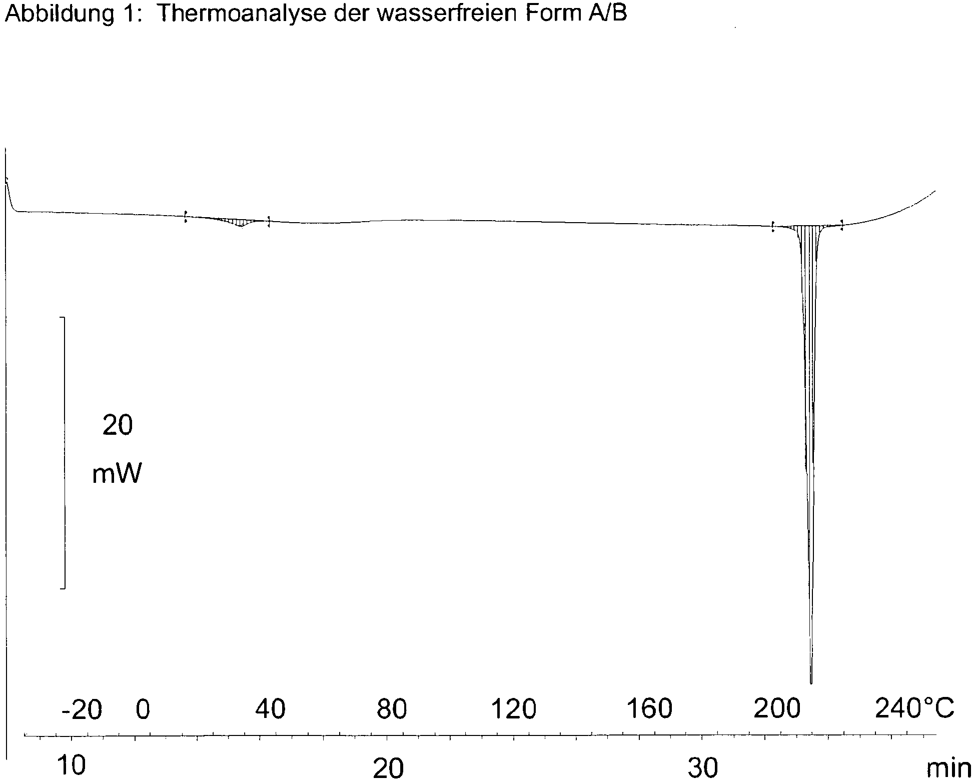

Polymorph A schmilzt bei 206 ± 3 °C. Im DSC-Diagramm ist ein weiteres schwach endothermes Signal bei ca. 25 °C zu beobachten. Hierbei handelt es sich um einen voll reversiblen Fest-Fest-Phasenübergang zwischen den beiden enantiotropen Kristallmodifikationen A und B. Die Form A ist oberhalb dieser Umwandlungstemperatur die thermodynamische stabile Modifikation, Form B ist unterhalb dieser Umwandlungstemperatur die thermodynamische stabile Modifikation.

Abbildung 2 zeigt ein cyclisches DSC-Diagramm, bei dem insgesamt 3 x über den Phasenübergang von -40 °C nach 120 °C und umgekehrt hinweg gefahren wurde. Beim Aufheizen wird dabei der Phasenübergang als endothermes Signal beobachtet, beim Abkühlen entsprechend als exothermes Signal. Beim ersten Aufheizzyklus kann der Phasenübergang auch als endothermes Doppelsignal bzw. als sehr breites Signal beobachtet werden, bei allen weiteren Zyklen tritt das Signal als sehr scharfes endothermes bzw. exothermes Signal auf, je nachdem ob aufgeheizt oder abgekühlt wird.

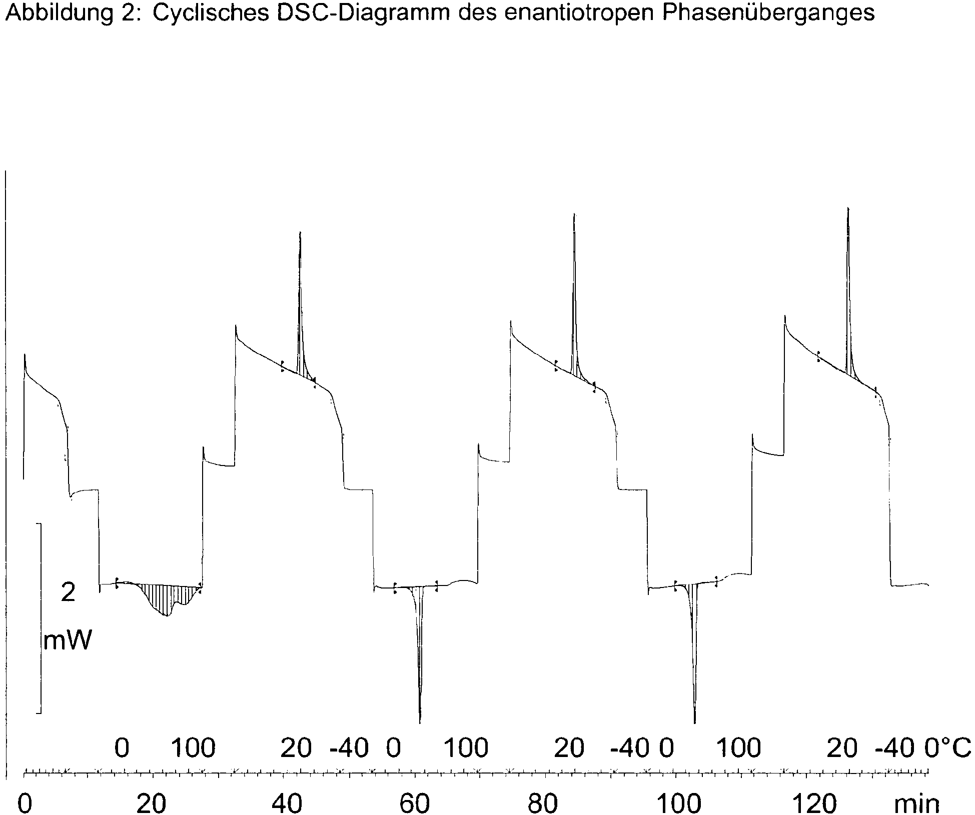

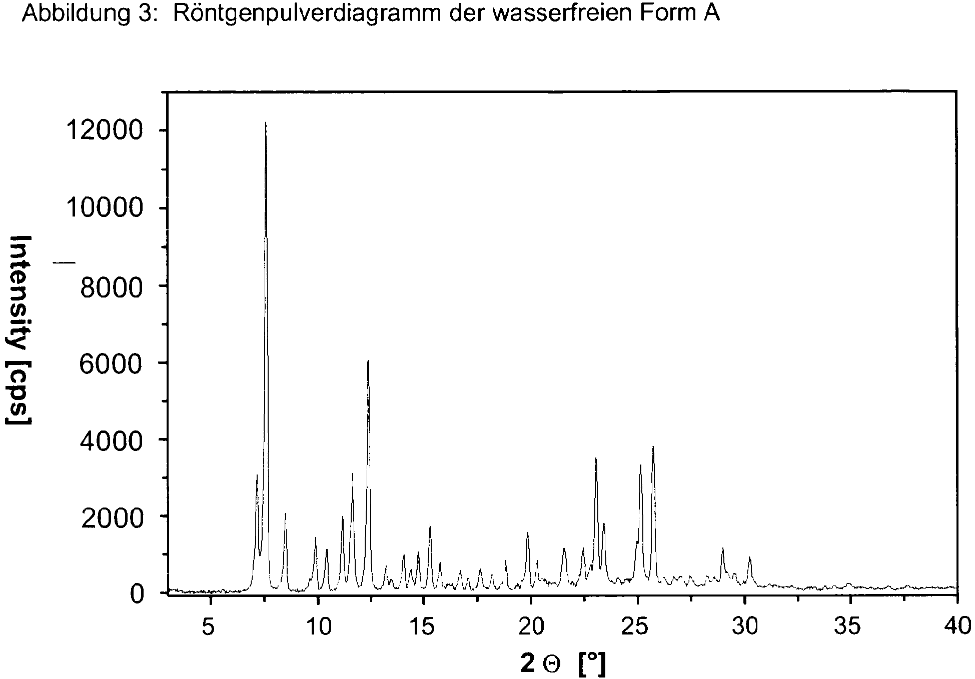

**Tabelle 1: Indizierte Röntgenreflexe bis 30 ° 2 Θ mit Intenistäten (normiert) für das wasserfreie Polymorph A**

| **2 Θ** | **Intensität** | **dₕₖₗ** | **Indizierung** | | | **d_{exp-calc}** |
|---|---|---|---|---|---|---|
| **[°]** | **I/Iₒ [%]** | **[Å]** | **h** | **k** | **l** | **[Å]** |
| 5,56 | 1 | 15,89 | 1 | 0 | 0 | -0,008 |
| 7,18 | 32 | 12,31 | 0 | 1 | 1 | 0,005 |
| 7,62 | 100 | 11,59 | 1 | 1 | 0 | 0,007 |
| 8,49 | 20 | 10,41 | -1 | 1 | 1 | 0,002 |
| 9,91 | 24 | 8,92 | 0 | 0 | 2 | 0,003 |
| 10,41 | 18 | 8,49 | 0 | 2 | 0 | 0,024 |
| 11,18 | 24 | 7,91 | 2 | 0 | 0 | 0,038 |
| 11,63 | 41 | 7,60 | -1 | 1 | 2 | 0,003 |
| 12,37 | 59 | 7,15 | -1 | 2 | 1 | -0,003 |
| 13,19 | 6 | 6,71 | 1 | 2 | 1 | -0,014 |
| 13,45 | 3 | 6,58 | -2 | 0 | 2 | 0,007 |
| 14,05 | 6 | 6,30 | 2 | 1 | 1 | 0,011 |
| 14,38 | 6 | 6,16 | 0 | 2 | 2 | 0,003 |
| 14,71 | 10 | 6,02 | -1 | 2 | 2 | -0,008 |
| 15,26 | 13 | 5,80 | 2 | 2 | 0 | 0,001 |
| 15,76 | 10 | 5,62 | -1 | 1 | 3 | 0,008 |
| 16,09 | 1 | 5,51 | 1 | 2 | 2 | -0,010 |
| 16,32 | 1 | 5,43 | 2 | 0 | 2 | 0,035 |
| 16,69 | 4 | 5,31 | 2 | 2 | 1 | -0,007 |
| 17,03 | 3 | 5,20 | -1 | 3 | 1 | 0,026 |
| 17,63 | 6 | 5,03 | 1 | 3 | 1 | 0,006 |
| 18,17 | 5 | 4,88 | -1 | 2 | 3 | -0,004 |
| 18,78 | 7 | 4,72 | -1 | 3 | 2 | -0,014 |
| 19,30 | 1 | 4,60 | -2 | 3 | 1 | -0,019 |
| 19,61 | 2 | 4,52 | -3 | 2 | 1 | 0,036 |
| 19,86 | 20 | 4,47 | -2 | 2 | 3 | 0,040 |
| 20,29 | 10 | 4,37 | 2 | 0 | 3 | 0,019 |
| 20,57 | 4 | 4,31 | 0 | 1 | 4 | 0,006 |
| 21,12 | 1 | 4,20 | 3 | 0 | 2 | 0,048 |
| 21,57 | 12 | 4,12 | -2 | 1 | 4 | 0,028 |
| 22,46 | 10 | 3,96 | 1 | 4 | 1 | 0,035 |
| 23,03 | 35 | 3,86 | 4 | 1 | 0 | 0,022 |
| 23,39 | 21 | 3,80 | -1 | 4 | 2 | 0,019 |
| 24,08 | 2 | 3,69 | -3 | 1 | 4 | -0,006 |
| 24,51 | 1 | 3,63 | -4 | 0 | 3 | 0,036 |
| 24,91 | 10 | 3,57 | -2 | 4 | 2 | 0,003 |
| 25,14 | 39 | 3,54 | 3 | 1 | 3 | 0,043 |
| 25,69 | 36 | 3,47 | -3 | 3 | 3 | 0,041 |
| 26,68 | 3 | 3,34 | 0 | 5 | 1 | 0,035 |
| 26,90 | 2 | 3,31 | 3 | 4 | 0 | 0,027 |
| 27,10 | 2 | 3,29 | 0 | 2 | 5 | 0,030 |
| 27,42 | 3 | 3,25 | 4 | 3 | 0 | 0,006 |
| 28,19 | 2 | 3,16 | -1 | 5 | 2 | -0,035 |
| 28,54 | 2 | 3,12 | 3 | 0 | 4 | 0,047 |
| 28,94 | 11 | 3,08 | 0 | 4 | 4 | -0,036 |
| 29,18 | 5 | 3,06 | -4 | 3 | 3 | 0,017 |
| 29,50 | 4 | 3,03 | -1 | 0 | 6 | 0,041 |
| 30,18 | 7 | 2,96 | -1 | 5 | 3 | -0,042 |

**Tabelle 2: Gittermetrik der wasserfreien Form A**

| Symmetrie: | Monoklin |
|---|---|
| Raumgruppe: | P |
| a: | 16,16(2) Å |
| b: | 17,02(1) Å |
| c: | 18,18(2) Å |
| ß: | 100,95(6)° |
| Zellvolumen: | 4907(11) Å³ |

### Beispiel 2 Kristallisation des Polymorphs B

Polymorph B erhält man, durch Abkühlen der Form A aus Beispiel 1 auf Temperaturen <10 °C.

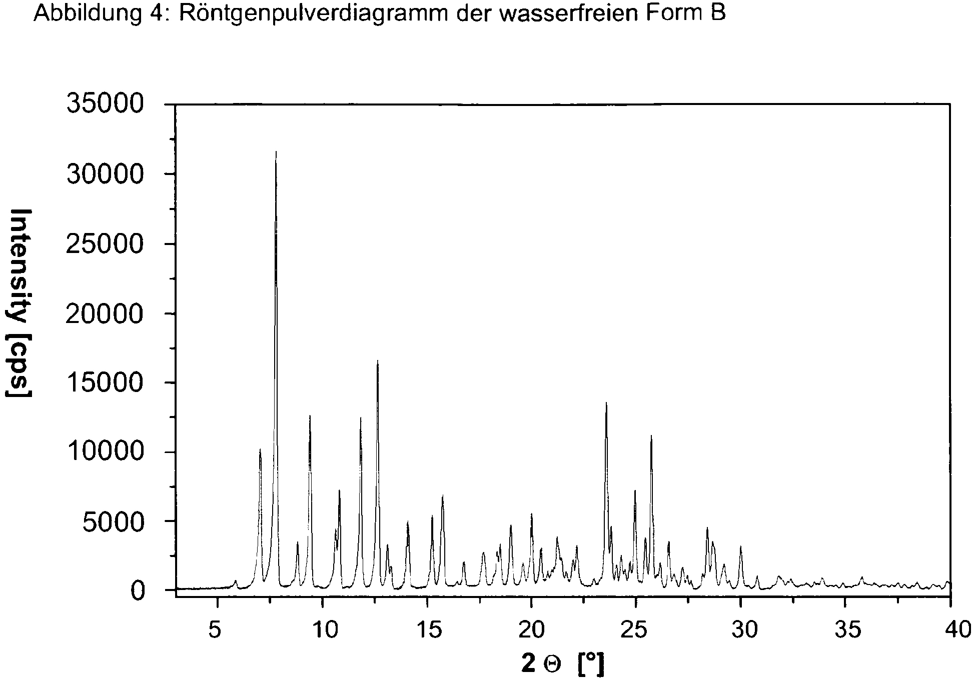

**Tabelle 3: Indizierte Röntgenreflexe bis 30 ° 2 Θ mit Intensitäten (normiert) für die wasserfreie Form B**

| **2 Θ** | **Intensität** | **dₕₖₗ** | **Indizierung** | | | **d_{exp-calc}** |
|---|---|---|---|---|---|---|
| **[°]** | **I/Iₒ [%]** | **[Å]** | **h** | **k** | **l** | **[Å]** |
| 5,82 | 3 | 15,17 | 1 | 0 | 0 | -0,007 |
| 7,04 | 33 | 12,55 | 0 | 1 | 1 | 0,001 |
| 7,82 | 100 | 11,3 | 1 | 1 | 0 | -0,004 |
| 8,84 | 11 | 10 | -1 | 1 | 1 | 0,001 |
| 9,44 | 40 | 9,36 | 1 | 1 | 1 | 0,011 |
| 10,62 | 14 | 8,32 | -1 | 0 | 2 | 0,013 |
| 10,79 | 24 | 8,19 | 0 | 1 | 2 | -0,005 |
| 11,82 | 39 | 7,48 | -1 | 1 | 2 | -0,003 |
| 12,64 | 53 | 7 | -1 | 2 | 1 | -0,009 |
| 13,07 | 11 | 6,77 | 1 | 2 | 1 | -0,006 |
| 13,24 | 6 | 6,68 | -2 | 1 | 1 | 0,004 |
| 14,04 | 16 | 6,3 | 2 | 1 | 1 | 0,003 |
| 15,23 | 17 | 5,81 | -2 | 1 | 2 | 0,003 |
| 15,70 | 22 | 5,64 | 2 | 2 | 0 | 0,016 |
| 16,38 | 2 | 5,41 | 0 | 3 | 1 | -0,010 |
| 16,73 | 6 | 5,3 | 2 | 2 | 1 | 0,008 |
| 17,67 | 8 | 5,02 | 0 | 2 | 3 | 0,014 |
| 18,16 | 3 | 4,88 | -1 | 2 | 3 | 0,005 |
| 18,33 | 9 | 4,84 | 3 | 1 | 0 | 0,016 |
| 18,48 | 10 | 4,8 | -3 | 1 | 1 | -0,003 |
| 18,97 | 15 | 4,68 | 0 | 0 | 4 | -0,001 |
| 19,56 | 6 | 4,54 | 1 | 3 | 2 | 0,013 |
| 20,00 | 17 | 4,44 | 2 | 1 | 3 | 0,000 |
| 20,42 | 9 | 4,35 | 1 | 0 | 4 | 0,009 |
| 20,76 | 4 | 4,27 | 3 | 0 | 2 | -0,014 |
| 20,97 | 4 | 4,23 | 0 | 4 | 0 | 0,010 |
| 21,07 | 5 | 4,21 | 1 | 1 | 4 | -0,009 |
| 21,22 | 12 | 4,18 | 0 | 3 | 3 | 0,001 |
| 21,40 | 7 | 4,15 | 3 | 2 | 1 | 0,004 |
| 21,66 | 4 | 4,1 | -1 | 3 | 3 | 0,018 |
| 21,98 | 7 | 4,04 | 2 | 2 | 3 | -0,003 |
| 22,16 | 10 | 4,01 | -3 | 1 | 3 | 0,008 |
| 22,97 | 3 | 3,87 | 1 | 2 | 4 | -0,006 |
| 23,58 | 43 | 3,77 | -2 | 3 | 3 | -0,003 |
| 23,78 | 15 | 3,74 | -2 | 2 | 4 | -0,004 |
| 24,05 | 6 | 3,7 | 4 | 1 | 0 | -0,002 |
| 24,29 | 8 | 3,66 | -2 | 4 | 1 | -0,008 |
| 24,46 | 5 | 3,64 | 3 | 3 | 1 | 0,018 |
| 24,71 | 7 | 3,6 | 0 | 3 | 4 | 0,001 |
| 24,96 | 23 | 3,56 | 2 | 3 | 3 | -0,001 |
| 25,45 | 12 | 3,5 | -2 | 4 | 2 | -0,010 |
| 25,75 | 35 | 3,46 | 4 | 2 | 0 | 0,011 |
| 25,99 | 4 | 3,43 | 3 | 2 | 3 | 0,014 |
| 26,15 | 6 | 3,41 | 3 | 3 | 2 | 0,010 |
| 26,57 | 12 | 3,35 | -2 | 3 | 4 | -0,001 |
| 26,82 | 4 | 3,32 | -3 | 2 | 4 | 0,011 |
| 27,20 | 6 | 3,28 | 1 | 2 | 5 | -0,010 |
| 27,43 | 4 | 3,25 | -2 | 4 | 3 | -0,003 |
| 27,60 | 3 | 3,23 | -2 | 2 | 5 | -0,005 |
| 28,19 | 4 | 3,16 | 3 | 4 | 1 | 0,010 |
| 28,40 | 15 | 3,14 | 0 | 4 | 4 | -0,013 |
| 28,64 | 12 | 3,11 | 0 | 0 | 6 | 0,016 |
| 29,18 | 6 | 3,06 | -4 | 3 | 2 | 0,004 |
| 29,42 | 2 | 3,03 | 1 | 4 | 4 | 0,002 |
| 29,99 | 10 | 2,98 | 0 | 5 | 3 | -0,008 |
| 30,77 | 3 | 2,9 | -4 | 3 | 3 | 0,018 |

**Tabelle 4: Gittermetrik der wasserfreien Form B**

| Symmetrie: | Monoklin |
|---|---|
| Raumgruppe: | P2₁/c (# 14) |
| a: | 15,23(1) Å |
| b: | 16,94(1) Å |
| c: | 18,79(1) Å |
| ß: | 95,6(2)° |
| Zellvolumen: | 4823(3) Å³ |

### Beispiel 3 Kristallisation des Polymorphs C

1-[(4-Methyl-chinazolin-2-yl)methyl]-3-methyl-7-(2-butin-1-yl)-8-(3-(R)-amino-piperidin-1-yl)-xanthin Rohsubstanz (26kg) wird mit 157 l Methanol zum Rückfluß erhitzt, mit 1,3 kg Aktiv-Kohle versetzt und nach 30 minütigem Rühren filtriert und mit 26 l Methanol nachgespült. Aus dem Filtrat werden 122 l Methanol abdestilliert, der Rückstand wird anschliessend 45-55°C abgekühlt. Zum Rückstand werden 52 l tert.-Butylmethylether über 30 Minuten hinzugegeben. Anschliessend wird weitere 60 Minuten bei 45-55°C nachgerührt. Innerhalb dieser Zeit erfolgt die Kristallisation. Zur Suspension werden weitere 78 l tert. Butylmethylether über 30 Minuten zugegeben und anschliessend wird noch einmal 60 Minuten bei 45-55°C nachgerührt. auf das vierfache Volumen verdünnt. Die Suspension wird langsam auf 15-25°C abgekühlt und über Nacht ei dieser Temperatur nachgerührt. Nach Abkühlen der Suspension auf 0-5°C werden die Kristalle abgesaugt, mit 2 Portionen tert.-Butylmethylether nachgewaschen und im Vakuumtrockenschrank bei 70°C getrocknet.

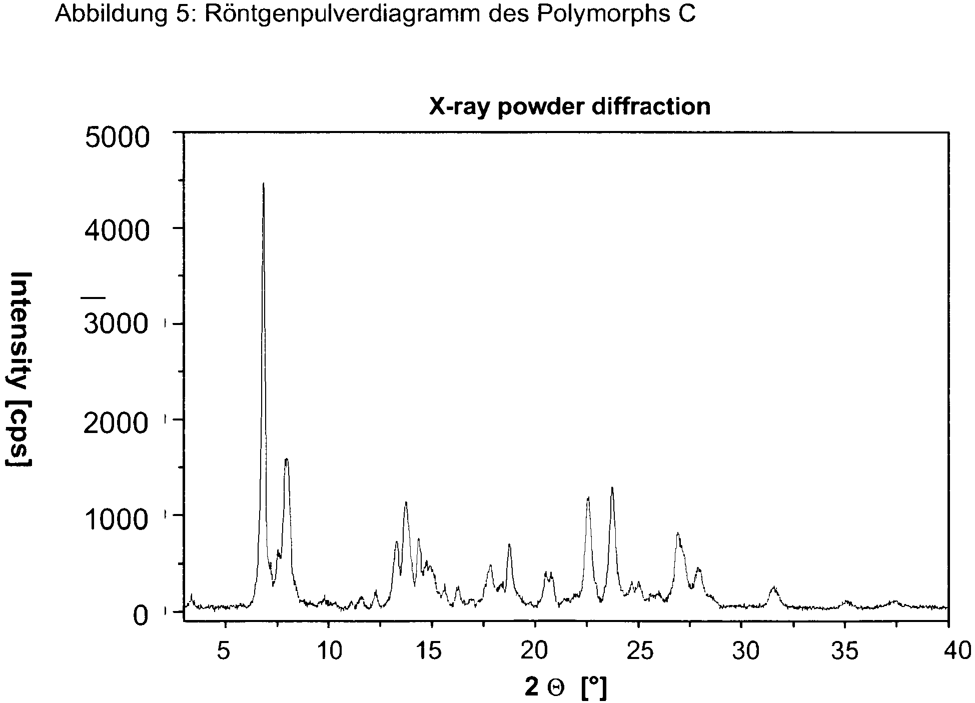

**Tabelle 5: Röntgenreflexe bis 30 ° 2 Θ mit Intensitäten (normiert) für die wasserfreie Form C**

| **2 Θ** | **dₕₖₗ** | **Intensität** | | **2 Θ** | **dₕₖₗ** | **Intensität** | | **2 Θ** | **dₕₖₗ** | **Intensität** |
|---|---|---|---|---|---|---|---|---|---|---|
| **[°]** | **[Å]** | **I/Iₒ [%]** | | **[°]** | **[Å]** | **I/Iₒ [%]** | | **[°]** | **[Å]** | **I/Iₒ [%]** |
| 3,38 | 26,16 | 4 | | 14,38 | 6,16 | 17 | | 21,96 | 4,05 | 4 |
| 6,85 | 12,90 | 100 | | 14,74 | 6,01 | 11 | | 22,59 | 3,93 | 26 |
| 7,18 | 12,31 | 11 | | 14,95 | 5,92 | 10 | | 23,76 | 3,74 | 29 |
| 7,52 | 11,74 | 14 | | 15,63 | 5,66 | 6 | | 24,68 | 3,60 | 6 |
| 7,96 | 11,10 | 36 | | 16,28 | 5,44 | 5 | | 25,01 | 3,56 | 7 |
| 9,80 | 9,02 | 3 | | 17,81 | 4,98 | 10 | | 25,57 | 3,48 | 4 |
| 11,11 | 7,96 | 2 | | 18,33 | 4,83 | 6 | | 25,96 | 3,43 | 4 |
| 11,58 | 7,64 | 3 | | 18,75 | 4,73 | 15 | | 26,93 | 3,31 | 18 |
| 12,30 | 7,19 | 5 | | 20,51 | 4,33 | 8 | | 27,22 | 3,27 | 13 |
| 13,30 | 6,65 | 16 | | 20,77 | 4,27 | 8 | | 27,92 | 3,19 | 10 |
| 13,75 | 6,44 | 26 | | 21,47 | 4,14 | 3 | | | | |

### Beispiel 4 Kristallisation des Polymorphs D

Polymorph D erhält man, wenn Polymorph C aus Beispiel 3 auf eine Temperatur von 30-100°C erwärmt bzw. bei dieser Temperatur trocknet wird.

### Beispiel 5 Kristallisation des Polymorphs E

Wasserfreies Polymorph E entsteht, wenn Polymorph D geschmolzen wird. Bei weiterer Erwärmung kristallisiert aus der Schmelze Polymorph E aus.

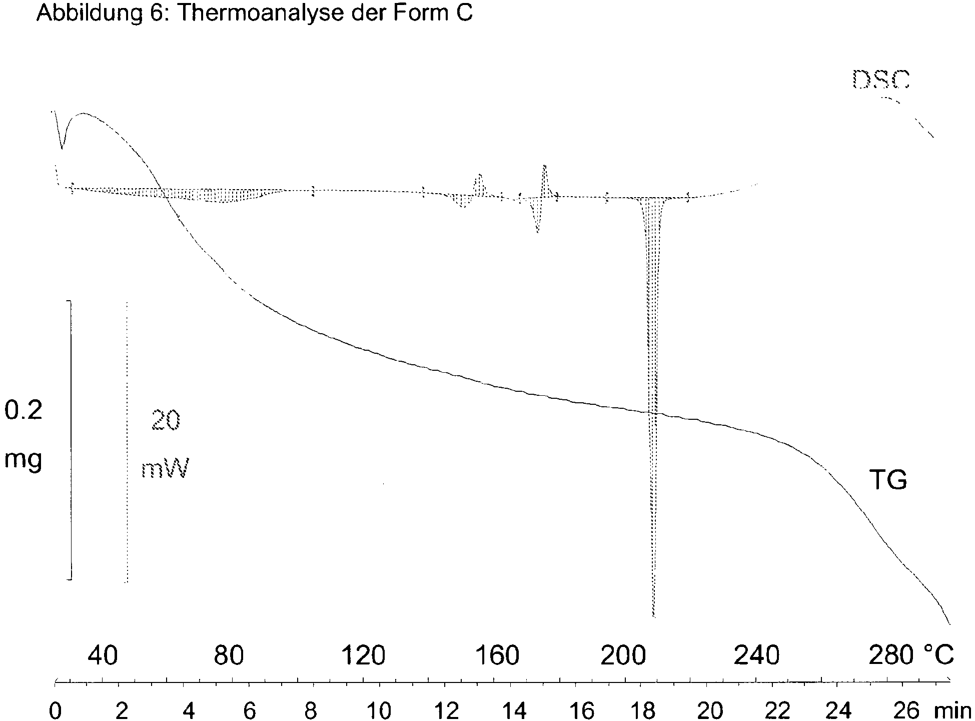

Im DSC-Diagramm der Form C sind eine ganze Reihe von Signalen zu beobachten. Stärkstes Signal ist der Schmelzpunkt der wasserfreien Form A bei ca. 206 °C, welche innerhalb des DSC-Experimentes erzeugt wird. Vor dem Schmelzpunkt sind eine Reihe weiterer endothermer und exothermer Signale zu beobachten. So zum Beispiel ein sehr breites und schwaches endothermes Signal zwischen 30 und 100°C, welches mit dem Hauptgewichtsverlust in der Thermogravimetrie (TR) korreliert. Aus einem TG/IR-Kopplungsexperiment kann die Information abgeleitet werden, dass nur Wasser aus der Probe in diesem Temperaturbereich entweicht. Ein Röntgenpulverdiagramm, welches von einer bei 100 °C getemperten Probe aufgenommen wurde, zeigt andere Röntgenreflexe als das Ausgangsmaterial, was den Schluß nahe legt, daß es sich bei Form C um eine Hydratphase handelt mit einer Stöchiometrie irgendwo im Bereich eines Hemihydrates oder Monohydrates. Bei der getemperten Probe handelt es sich um eine weitere wasserfreie Modifikation D, die allerdings nur unter wasserfreien Bedingungen stabil ist. Die Form D schmilzt bei ca. 150 °C. Aus der Schmelze kristallisiert eine weitere wasserfreie Kristallmodifikation E, die bei weiterer Erwärmung bei ca. 175 °C schmilzt. Aus der Schmelze der Form E kristallisiert letztendlich Form A. Form E ist ebenfalls eine metastabile Kristallmodifikation, die nur bei hohen Temperaturen auftritt.

## Patentansprüche

**1.** Wasserfreies Polymorph A der Verbindung 1-[(4-Methyl-chinazotin-2-yl)methyl]-3-methyl-7-(2-butin-1-yl)-8-(3-(R)-amino-piperidin-1-yl)-xanthin **dadurch gekennzeichnet, dass** es bei 206±3 °C schmilzt.

**2.** Polymorph A nach Anspruch 1, **dadurch gekennzeichnet, daß** es im Röntgenpulverdiagramm unter anderen charakteristische Reflexe bei folgenden d-Werten aufweist: 11.49 Å, 7.60 Å, 7.15 Å, 3.86 Å, 3.54 Å und 3.47.

**3.** Wasserfreies Polymorph B der Verbindung 1-[(4-Methyl-chinazolin-2-yl)methyl]-3-methyl-7-(2-butin-1-yl)-8-(3-(R)-amino-piperidin-1-yl)-xanthin **dadurch gekennzeichnet, dass** es sich bei einer Temperatur von 10-40°C reversibel in das Polymorph A von Anspruch 1 umwandelt.

**4.** Polymorph B nach Anspruch 3, **dadurch gekennzeichnet, daß** es im Röntgenpulverdiagramm unter anderen charakteristische Reflexe bei folgenden d-Werten aufweist: 11.25 Å, 9.32 Å, 7.46 Å, 6.98 Å und 3.77 Å.

**5.** Polymorph C der Verbindung 1-[(4-Methyl-chinazolin-2-yl)methyl]-3-methyl-7-(2-butin-1-yl)-8-(3-(R)-amino-piperidin-1-yl)-xanthin **dadurch gekennzeichnet, dass** es bei einer Temperatur von 30-100°C Wasser verliert und im DSC-Diagramm bei ca. 150°C und 175°C weitere thermische Effekte zeigt.

**6.** Polymorph C nach Anspruch 5, **dadurch gekennzeichnet, daß** es im Röntgenpulverdiagramm unter anderen charakteristische Reflexe bei folgenden d-Werten aufweist: 12.90 Å, 11.10 Å, 6.44 Å, 3.93 Å und 3.74 Å.

**7.** Wasserfreies Polymorph D der Verbindung 1-[(4-Methyl-chinazolin-2-yl)methyl]-3-methyl-7-(2-butin-1-yl)-8-(3-(R)-amino-piperidin-1-yl)-xanthin **dadurch gekennzeichnet, dass** es bei 150±3 °C schmilzt.

**8.** Wasserfreies Polymorph E der Verbindung 1-[(4-Methyl-chinazolin-2-yl)methyl]-3-methyl-7-(2-butin-1-yl)-8-(3-(R)-amino-piperidin-1-yl)-xanthin **dadurch gekennzeichnet, dass** es bei 175±3 °C schmilzt.

**9.** Verfahren zur Herstellung des Polymorphs C nach Anspruch 5 **dadurch gekennzeichnet, dass** man
(a) 1-[(4-Methyl-chinazolin-2-yl)methyl]-3-methyl-7-(2-butin-1-yl)-8-(3-(R)-amino-piperidin-1-yl)-xanthin in Methanol zum Rückfluß erhitzt,
(b) die methanolische Lösung auf eine Temperatur von 40-60°C abkühlt,
(c) mit einem Lösungsmittel wie tert.-Butylmethylether versetzt,
(d) die resultierende Suspension zunächst auf 15-25°C und anschliessend auf 0-5°C abkühlt,
(e) die Kristalle absaugt und
(f) bei einer Temperatur von 70°C im Vakuum trocknet.

**10.** Verfahren nach Anspruch 9 **dadurch gekennzeichnet, dass** man im Anschluss an Schritt (a) die heiße Lösung filtriert.

**12.** Verwendung eines der Polymorphe A, B, oder C der Verbindung 1-[(4-Methyl-chinazolin-2-yl)methyl]-3-methyl-7-(2-butin-1-yl)-8-(3-(R)-amino-piperidin-1-yl)-xanthin zur Herstellung eines Arzneimittels für die Behandlung von Patienten mit Diabetes mellitus Typ I und Typ II, Prädiabetes oder verminderter Glukosetoleranz, rheumatoider Arthritis, Adipositas, oder durch Calcitonin verursachter Osteoporose sowie von Patienten, bei denen eine Allograft Transplantation vorgenommen wurde.

**13.** Arzneimittel enthaltend die Verbindung 1-[(4-Methyl-chinazolin-2-yl)methyl]-3-methyl-7-(2-butin-1-yl)-8-(3-(R)-amino-piperidin-1-yl)-xanthin neben einem oder mehreren inerten Trägerstoffen und/oder Verdünnungsmitteln **dadurch gekennzeichnet, dass** es wenigstens 0,1% bis 0,5% eines der Polymorphe A, B, oder C enthält.
